# EUROPEAN PATENT SPECIFICATION

(11) **EP 0 354 992 B1**
(45) Date of publication and mention of the grant of the patent: **22.03.1995**
(21) Application number: 89112463.8
(22) Date of filing: 07.07.1989
(51) Int. Cl.: A61K 38/00

(54) **Conjugates of VIP and active fragments thereof with hydrophobic moieties and topical compositions for use in the treatment of male impotence**
VIP-Konjugate und deren aktive Fragmente mit hydrophoben Anteilen und topische Zuberetungen zur Verwendung in der Behandlung männlicher Impotenz
Conjugués de VIP et de ses fragments actifs avec des portions hydrophobiques et des préparations pour l'utilisation dans le traitement de l'impuissance masculine

(30) Priority: 08.07.1988 IL 87055
(43) Date of publication of application: 21.02.1990
(73) Proprietor: YEDA RESEARCH AND DEVELOPMENT COMPANY LIMITED, Rehovot 76100 (IL)
(72) Inventor: Gozes, Illana, Ness Ziona (IL); Fridkin, Matityahu, Rehovot (IL)
(74) Representative: VOSSIUS & PARTNER

(56) References cited:
- EP-A- 0 184 309
- EP-A- 0 225 639
- EP-A- 0 241 926
- EP-A- 0 297 068
- US-A- 3 879 371
- US-A- 4 065 641
- BIOLOGICAL ABSTRACT, no. 35007263; A. SIDI et al.: "Patient acceptance of andsatisfaction with vasoactive intracavernous ..." & J. Urol. 1988, vol. 139, no.4, part 2, page 328A
- BIOLOGICAL ABSTRACT, no. 33042928; A. SIDI et al.: "Short and long termcomplication of vasoactive intracavernous..." & J. Urol. 1987, vol. 137, no. 4,part 2, page 203A
- PATENT ABSTRACTS OF JAPAN, vol. 6, no. 3 (C-86)(881), 9 January 1982; & JP-A-56128721 (EISAI) 08.10.1981
- PATENT ABSTRACTS OF JAPAN vol. 10, no. 303 (C-378)(2359), 16 October 1986; &JP-A-61118399 (OTSUKA) 05.06.1986

## Description

The invention relates to pharmaceutical compositions for the treatment of male impotence.

Impotence, a condition that can bring much suffering to the life of the afflicted individual and those surrounding him, can be caused by psychological as well as organic problems. It is estimated that in the United States alone, 10 million men suffer from varying degrees of impotence. In general, impotence is a predominant syndrome affecting at least 10-15% of the male population. In men above 40 years of age, the occurrence of impotence is prevalent due to neuroendocrine failures associated with aging, and any man over the age of 40 years may experience occasional impotence. The mechanism of penile erection is a complicated one and requires the intactness of the endocrine system, the nervous system and the vascular system. Many times the suffering individual is hesitant to seek medical help and at the moment the remedies available to the physician are rather limited. Organic abnormalities can be treated by surgery and implantations. Erection was also obtained by injection of smooth muscle relaxants such as phenoxybenzamine. It is, of course, quite understandable that superior treatment is needed and the invention relates to an ointment to treat impotence.

It is the object of the invention to provide an improved product for the treatment of male impotence. This object is solved by the surprising finding that the transdermal application of vasoactive intestinal peptide (VIP) or derivatives or fragments thereof, coupled to a suitable hydrophobic moiety, enhances sexual activity.

Therefore, the subject matter of the invention is a conjugate of vasoactive intestinal peptide (VIP), an active derivative or fragment thereof, coupled to a hydrophobic moiety and a transdermally effective pharmaceutical composition for topical application for the treatment of male impotence which comprises the conjugate as active ingredient.

The active ingredients are VIP, functional derivatives thereof, active fragments thereof, and modified peptides wherein one or more of the amino acids are substituted by others. The backbone of the compositions is a naturally occuring material, or derivative thereof.

The peptide of interests, Vasoactive Intestinal Peptide (VIP) fulfils several of the chemical criteria for a neurotransmitter in penile erection in the male: it is present in nerve fibers with nerve endings around cavernous smooth muscle and blood vessels and it is elevated during erection. Moreover, injection of exogenous VIP induces erection in man. It is important to add that in impotent men it was unequivocally shown that VIP quantities decrease locally in their penises. Since VIP was found to be a key neurotransmitter in erection formation, its local administration should help to relieve the dysfunction. Past studies used local VIP injection into the penis as the mode of drug administration. Using an animal model, we developed an ointment containing modified VIP which enhances mating behaviour in mammals, as evident from experiments with rats.

The behavioural model for studies of sexual behaviour was the castrated rat model. Castrated rats lose their sexual activity in a time dependent manner and we used this characteristic to develop a model for investigating the therapeutic activity of VIP.

The sequence of VIP, a 29-amino acid peptide, is as follows: 
1                                   7
H-His-Ser-Asp-Ala-Val-Phe-Thr-Asp-Asn-Tyr-Thr-Arg-

              16
-Leu-Arg-Lys-Gln-Met-Ala-Val-Lys-Lys-Tyr-Leu-Asn-

              28
-Ser-Ile-Leu-Asn-NH₂
The peptide chain was assembled according to the solid-phase methodology (Merrifield (1963) J. Am. Chem. Soc. 85:2149). Partial sequences were also synthesized by this approach. VIP₇₋₂₈ and VIP₁₆₋₂₈, for example, are active fragments.

The following hydrophobic derivatives of VIP were prepared by attaching, as the last coupling step, a long chain aliphatic carboxylic acid to the N-terminus of the peptide chain. This was accomplished while the peptide was still attached to the polymeric support:
1. CH₃(CH₂)₆CO-VIP
2. CH₃(CH₂)₁₆CO-VIP
3. CH₃(CH₂)₁₆CO-VIP₇₋₂₈
4. CH₃(CH₂)₁₆CO-VIP₁₆₋₂₈
5. 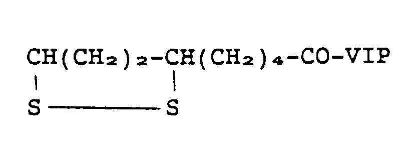
The following peptides were prepared by aminolysis of peptide-polymer conjungates with long-chain aliphatic amines; chain extension occurs at the C-terminus of the VIP:
1. VIP-NH CH₂CH₃
2. VIP-NH(CH₂)₃CH₃
3. VIP-NH(CH₂)₇CH₃
All peptides were purified by HPLC and characterized by their amino acid analysis.

In general, a conjugate of VIP, or an active fragment thereof, said active fragment having the activity that injection of such fragment induces erection in a man, is coupled to a hydrophobic moiety. The hydrophobic moiety may be an aliphatic moiety, such as a moiety of the formula CH₃(CH₂)ₙ-CO- where n is an integer from 1 to 16, or a moiety of the formula 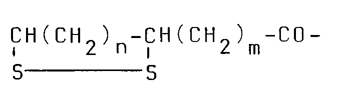
wherein n is an integer of from 2 to 6 and m is an integer of from 2 to 12, attached to the N-terminus of the VIP molecule, or an active fragment thereof, by coupling the corresponding carboxylic acids. The hydrophobic moiety may also be an aliphatic moiety, such as a moiety of the formula CH₃(CH₂)ₘ-NH- wherein m is an integer of from 1 to 12, attached to the C-terminus of the VIP molecule, or an active fragment thereof, by coupling the corresponding amines.

### Examples

### Synthesis of Stearyl-VIP

Synthesis was carried out via the solid-phase strategy. All amino acid derivatives were purchased from Peptide Institute Inc. (Japan). It was performed either automatically using Peptide Synthesizer (Applied Biosystem) or manually. α-Amino functional groups were protected by t-butyloxycarbonyl (t-Boc). Side chain protecting groups were as follows: His, N^{im}-p-toluenesulfonyl; Lys, 2-chlorobenzyloxycarbonyl;p Tyr, 02.6-dichlorobenzyl; Arg, N^{G}-p-toluenesulfonyl; Asp, β-benzyl; Thr and Ser, O-benzyl. The first amino acid (Asn) was attached to benzhydryl amine resin (Chemalog) using N.N'-dicyclohexylcarbodiimide (DCC) as a coupling agent. All coupling stages were performed with a threefold excess of protected amino acid derivatives using DCC and 1-hydroxylbenzotriazole (HOBt) as coupling agents. All stages of coupling were monitored with ninhydrin. The stearic acid residue was added similarly to amino acid residues using DCC/HOBt. Final cleavage of the peptide from the polymeric support along with deprotection was achieved with anhydrous HF. The product was first purified by gel-filtration on a Sephadex® G-25 column followed by HPLC on reversed phase C₁₈-column using a linear gradient obtained by mixing (A) 0.1% trifluoroacetic acid (TFA) in water and (B) 0.1% TFA in 70% acetonitrile and 30% water. The product gave, after acidic hydrolysis, the expected ratio of its constituent amino acids.

Other peptides were similarly prepared.

It is clear that introduction of hydrophobic moieties into the peptide chain of VIP can also take place within the chain, e.g., at the amino side chains of Lys. The hydrophobic moieties can be aliphatic, linear saturated (as in the compounds prepared), linear unsaturated (containing double or triple bonds), branched, saturated and unsaturated, linear and branched containing heteroatoms, aromatic-aliphatic or aromatic, and can be introduced into either C, N terminus or within the peptide chain, or at two or more positions. All these modifications can be performed on fragments of VIP as well, or on any other biologically active analog of VIP.
The animal model All tested rats (Wistar derived from the departmental colony, Hormone Research Department, at the Weizmann Institute of Science), are sexually experienced male rats around 3 months of age (body weight ∼250 g). At this age, the rats are castrated by removal of the testes and injected daily with testosterone for at least 14 consecutive days.

All rats (males and females) that take part in the experiment are kept in a 12-hour light 12-hour dark cycle. Prior to testing, each male is put in a separate cage for at least 1 hour. A sexually receptive female is introduced to each male. Females were tested for their receptivity by vaginal smearings. The latencies of mounts and intromissions are recorded. The tests are terminated if the male does not mount within 15 minutes. Using castrated animals we achieved decreased sexual activity which is expressed by longer intervals between intromissions and less intromissions during the 15 min observation periods.

Initially, we tested a few VIP analogs by injection (intravenously 5-10 »g/animal). As a control we injected saline at identical volumes (5-10 »l).

Table I shows the results for stearyl-VIP injection. At the upper left side of the chart we see that stearyl-VIP potentiates sexual activity in a comparable manner to VIP (right hand side of the table). This analog is lipophylic and hence may be a candidate for topical application.

As we have obtained positive results with stearyl-VIP by injection we have now developed a topical application method. This was achieved by mixing 20 »l of 1 »g/ml VIP-stearyl with 10 »l dimethyl sulfoxide (DMSO) and direct topical application on the sex organ. In Table II one can easily notice a remarkable sexual arousal after stearyl-VIP application compared with control rats subjected to DMSO. We therefore suggest that stearyl-VIP could be used as a potentiator of sexual activity in animals.

In Table III we compare the topical application of the carrier (DMSO) to stearic acid by itself, to VIP by itself and to stearyl-VIP. It is self-evident that stearyl-VIP is the most active analog. A shorter (8 amino acid) VIP analog which is related to the human immunodeficiency virus (HIV) peptide T (HIV-PEPT) which did not show much activity upon topical application by itself is somewhat active in a conjugated form but not as VIP-stearyl.
(A)The effect of topical application of stearyl-VIP on the sexual behaviour of male rats is summarized in Table IV.
   Preparation of Ointment - Lanolin (1 g), water (3 g) DMSO (7 drops) and 10 mg of stearyl-VIP were manually homogenized in a mortar. An amount of paste, containing 30-50 »g peptide was applied onto the rat's penis and sexual behaviour, following the methodology previously described, was monitored. As shown in the Table, the peptide showed about 25% decrease in intromission latency and two-fold increase in ejaculations in 10 animals.
(B) Using the same protocol as in (A) an ointment of VIP-oleyl was prepared and tested. It did show a certain effect in shortening of intromission latency (∼10%) and also increased, though much less than stearyl-VIP, the number of ejaculations.

The effective dose for humans is between 1 and 5 »g.

We claim that VIP-stearyl can be used in topical application to improve mammalian sexual behaviour in sexually deficient males. Moreover, we claim that further modification using the same idea of a lypophylic derivative may even improve the material as VIP is known to be a bronchodilator as well as a vasodilator. Other therapeutical applications such as in asthma and blood pressure regulation are also claimed.

### Legends to tables

### Table I

Rats were castrated and injected daily for 15 consecutive days with testosterone at 4 »g/100 g body weight (BW). Tests were performed with stearyl-VIP or VIP at 10 »g/animal.

### Table II

Rats were castrated and injected daily for 16 consecutive days with testosterone at 2 »g/100 g BW. Testing was performed by topical application of stearyl-VIP-20 »g/20 »l mixed with 10 »l DMSO and compared to topical application of 30 »l DMSO.

### Table III

6 rats, 120 days old, sexually experienced, were tested. The rats were castrated 14 days earlier and were injected with 4 »g/100 g BW of testosterone daily. In the table we see an increase in number of intromissions and shortening of latency after topical application of HIV-PEPT-stearyl and stearyl-VIP. All 6 materials were tested by topical application. Also there is a clear increase in the number of ejaculations after applications of HIV PEPT-stearyl and stearyl-VIP. Corresponding results are expected with human males.

### Table IV

Summary of the effect of topical application of stearyl-VIP on the sexual behaviour of male rats.

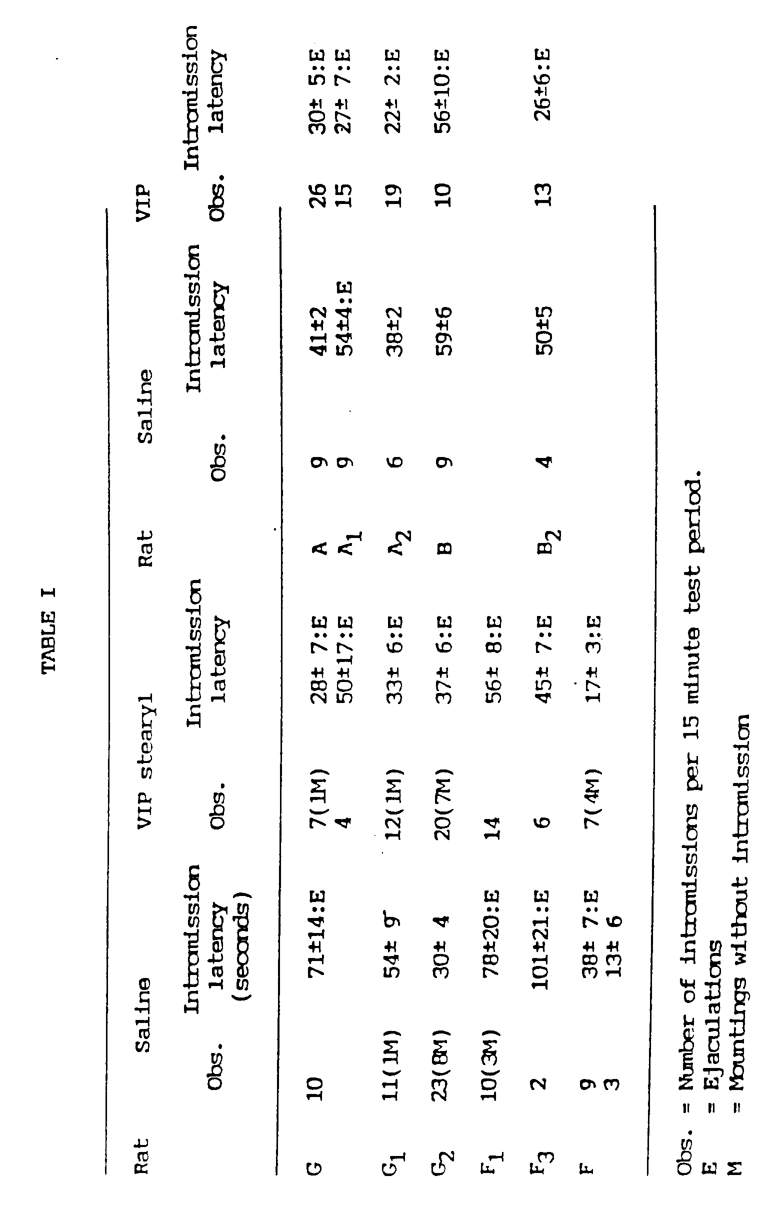

**TABLE II**

| TOPICAL APPLICATION | | | | |
|---|---|---|---|---|
| Rat | DMSO | | Stearyl-VIP | |
| | Obs. | Intromission latency | Obs. | Intromission latency |
| A₁ | 11 | 48±12 | 21 | 23±4 |
| A₂ | 8 | 79±30 | 14 | 29±4 |
| A₃ | - | - | 14 | 32±8 |
| B | 21 | 31±5:E | 10 | 16±3:E |
| | | | 3 | 38±10 |
| B₂ | - | - | 11(3M) | 18±7 |
| B₃ | 12 | 54±12 | 25 | 24±4 |
| Please rote that rats B₂ and A₃ were sexually active only after Stearyl-VIP application. | | | | |

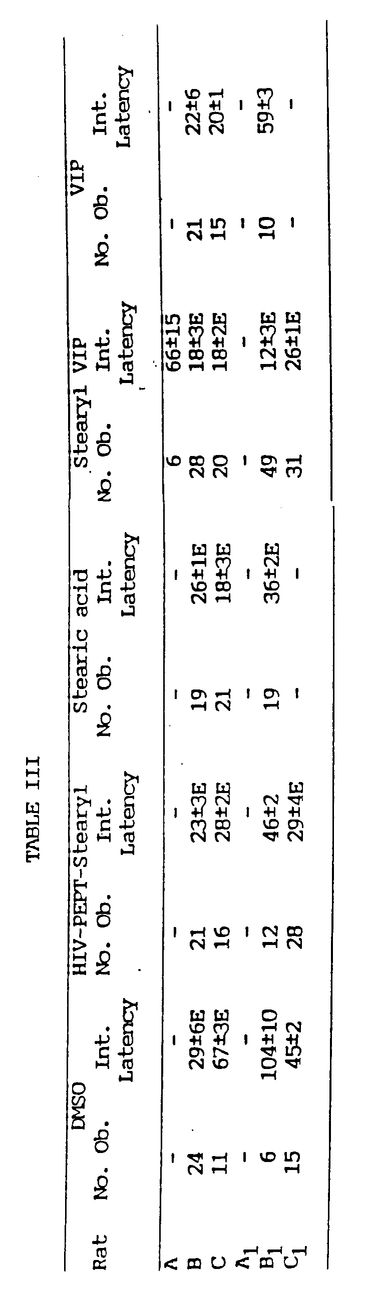

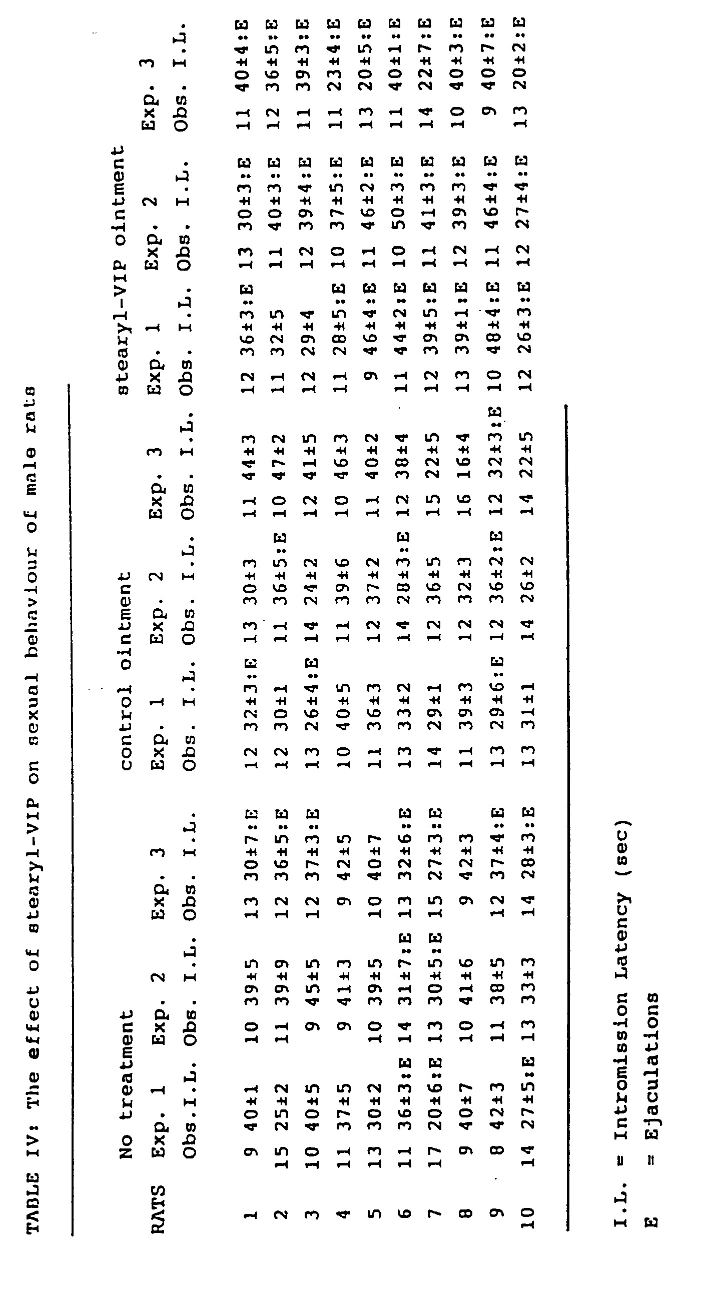

## Claims (Claims for the following Contracting State(s): AT, BE, CH, DE, FR, GB, GR, IT, LI, LU, NL, SE)

1. A conjugate of vasoactive intestinal peptide (VIP), or of an active fragment thereof, said active fragment having the activity that injection of such fragment induces erection in a man, coupled to a hydrophobic moiety.

2. A conjugate according to claim 1 wherein the hydrophobic moiety is an aliphatic moiety or a moiety of the formula 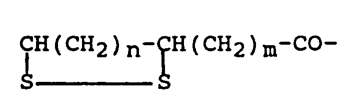 wherein n is an integer of from 2 to 6 and m is an integer of from 2 to 12, and is attached to the N-terminus of the VIP molecule or an active fragment thereof.

3. A conjugate according to claim 1 or 2 wherein a hydrophobic moiety is a moiety of the formula CH₃(CH₂)ₙ-CO-, where n is an integer of from 1 to 16, attached to the N-terminus of the VIP molecule or an active fragment thereof.

4. A conjugate according to claim 1 wherein the hydrophobic moiety is a moiety of the formula CH₃(CH₂)ₘ-NH-, wherein m is an integer of from 1 to 12, attached to the C-terminus of the VIP molecule or an active fragment thereof.

5. The conjugate of any one of claims 1 to 4 wherein the active fragment of VIP is VIP₇₋₂₈ or VIP₁₆₋₂₈.

6. The conjugate of claim 1 wherein the conjugate is represented by the formula
CH₃(CH₂)₆-CO-VIP or 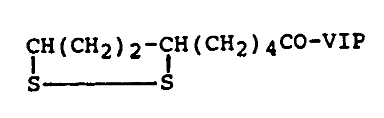
wherein the hydrophobic moiety is coupled through the N-terminus of the VIP, or active fragment thereof, or
VIP-NHCH₂CH₃
VIP-NH(CH₂)₃CH₃ or
VIP-NH(CH₂)₇CH₃
wherein the hydrophobic moiety is coupled through the C-terminus of the VIP.

7. The conjugate of claim 1 having the formula

CH3(CH2)16-CO-His-Ser-Asp-Ala-Val-Phe-Thr-
Asp-Asn-Tyr-Thr-Arg-Leu-Arg-Lys-Gln-Met-Ala-
Val-Lys-Lys-Tyr-Leu-Asn-Ser-Ile-Leu-Asn-NH2,

CH3(CH2)16-CO-Thr-Asp-Asn-Tyr-Thr-Arg-Leu-
Arg-Lys-Gln-Met-Ala-Val-Lys-Lys-Tyr-Leu-Asn-
Ser-Ile-Leu-Asn-NH2, or

CH3(CH2)16-CO-Gln-Met-Ala-Val-Lys-Lys-Tyr-
Leu-Asn-Ser-Ile-Leu-Asn-NH2.

8. A transdermal pharmaceutical composition useful for the treatment of male impotence comprising a physiologically effective quantity of a conjugate according to any one of claims 1 to 7 combined with a pharmaceutically acceptable carrier.

9. A composition according to claim 8 wherein the conjugate is stearyl-VIP and the carrier comprises DMSO.

10. A process for the preparation of a conjugate of vasoactive intestinal peptide (VIP), or an active fragment thereof, said active fragment having the activity that injection of such fragment induces erection in a man, coupled to a hydrophobic moiety, which comprises coupling a long chain aliphatic carboxylic acid to the N-terminus of the peptide chain or coupling a long-chain aliphatic amine to the C-terminus of the peptide chain.

11. The process of claim 10 wherein the aliphatic carboxylic acid is CH₃(CH₂)₁₆-COOH and the conjugate is one having the formula:

CH3(CH2)16-CO-His-Ser-Asp-Ala-Val-Phe-Thr-
Asp-Asn-Tyr-Thr-Arg-Leu-Arg-Lys-Gln-Met-Ala-
Val-Lys-Lys-Tyr-Leu-Asn-Ser-Ile-Leu-Asn-NH2,

CH3(CH2)16-CO-Thr-Asp-Asn-Tyr-Thr-Arg-Leu-
Arg-Lys-Gln-Met-Ala-Val-Lys-Lys-Tyr-Leu-Asn-
Ser-Ile-Leu-Asn-NH2, or

CH3(CH2)16-CO-Gln-Met-Ala-Val-Lys-Lys-Tyr-
Leu-Asn-Ser-Ile-Leu-Asn-NH2.

12. The process of claim 10 or 11 wherein the coupling of the aliphatic carboxylic acid to the N-terminus of the peptide chain comprises coupling stearic acid to the N-terminus of the peptide chain using N,N'-dicyclohexyl-carbodiimide and 1-hydroxylbenzotriazole as coupling agents.

## Claims (Claims for the following Contracting State(s): ES)

1. A process for the preparation of a conjugate of vasoactive intestinal peptide (VIP), or an active fragment thereof, said active fragment having the activity that injection of such fragment induces erection in a man, coupled to a hydrophobic moiety, which comprises coupling a long chain aliphatic carboxylic acid to the N-terminus of the peptide chain or coupling a long-chain aliphatic amine to the C-terminus of the peptide chain.

2. The process according to claim 1 wherein the aliphatic carboxylic acid has the formula CH₃(CH₂)ₙ-COOH, where n is an integer from 1 to 16.

3. The process according to claim 1 wherein the aliphatic amine is an amine of the formula CH₃(CH₂)ₘNH₂, wherein m is an integer from 1 to 12.

4. The process of any one of claims 1 to 3 wherein the active fragment of VIP is VIP₇₋₂₈ or VIP₁₆₋₂₈.

5. The process of claim 1 wherein the aliphatic carboxylic acid is CH₃(CH₂)₆-COOH and the conjugate is CH₃(CH₂)₆CO-VIP.

6. The process of claim 1 wherein the aliphatic carboxylic acid is CH₃(CH₂)₁₆-COOH and the conjugate is one having the formula:

CH3(CH2)16-CO-His-Ser-Asp-Ala-Val-Phe-Thr-
Asp-Asn-Tyr-Thr-Arg-Leu-Arg-Lys-Gln-Met-Ala-
Val-Lys-Lys-Tyr-Leu-Asn-Ser-Ile-Leu-Asn-NH2,

CH3(CH2)16-CO-Thr-Asp-Asn-Tyr-Thr-Arg-Leu-
Arg-Lys-Gln-Met-Ala-Val-Lys-Lys-Tyr-Leu-Asn-
Ser-Ile-Leu-Asn-NH2, or

CH3(CH2)16-CO-Gln-Met-Ala-Val-Lys-Lys-Tyr-
Leu-Asn-Ser-Ile-Leu-Asn-NH2.

7. The process of any one of claims 1, 2, 4 or 6 wherein the coupling of the aliphatic carboxylic acid to the N-terminus of the peptide chain comprises coupling stearic acid to the N-terminus of the peptide chain using N,N'-dicyclohexylcarbodiimide and 1-hydroxylbenzotriazole as coupling agents.

8. A process for the preparation of a transdermal pharmaceutical which comprises combining a physiologically effective quantity of a conjugate prepared according to any one of claims 1 to 7 with a pharmaceutically acceptable carrier.

9. The process of claim 8 wherein the conjugate is stearyl-VIP and the carrier comprises DMSO.

## Patentansprüche (Patentansprüche für folgende(n) Vertragsstaat(en): AT, BE, CH, DE, FR, GB, GR, IT, LI, LU, NL, SE)

1. Konjugat des vasoaktiven intestinalen Peptids (VIP) oder eines wirksamen Fragments davon, gekoppelt an eine hydrophobe Einheit, wobei das wirksame Fragment derart wirksam ist, daß die Injektion des Fragments eine Erektion beim Mann induziert.

2. Konjugat nach Anspruch 1, wobei die hydrophobe Einheit eine aliphatische Einheit oder eine Einheit der Formel 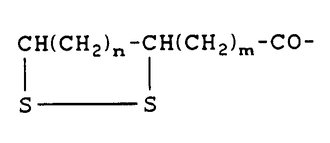 ist, in der n eine ganze Zahl von 2 bis 6 ist, und m eine ganze Zahl von 2 bis 12 ist, und an das N-Ende des VIP-Moleküls oder eines wirksamen Fragments davon gebunden ist.

3. Konjugat nach Anspruch 1 oder 2, wobei die hydrophobe Einheit eine Einheit der Formel CH₃(CH₂)ₙ-CO- ist, wobei n eine ganze Zahl von 1 bis 16 ist, die an das N-Ende des VIP-Moleküls oder eines wirksamen Fragments davon gebunden ist.

4. Konjugat nach Anspruch 1, wobei die hydrophobe Einheit eine Einheit der Formel CH₃(CH₂)ₘ-NH- ist, wobei m eine ganze Zahl von 1 bis 12 ist, die an das C-Ende des VIP-Moleküls oder eines wirksamen Fragments davon gebunden ist.

5. Konjugat nach einem der Ansprüche 1 bis 4, wobei das wirksame Fragment des VIP's VIP₇₋₂₈ oder VIP₁₆₋₂₈ ist.

6. Konjugat nach Anspruch 1, wobei das Konjugat durch die Formel
CH₃(CH₂)₆-CO-VIP oder 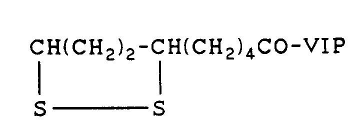
in der die hydrophobe Einheit durch das N-Ende des VIP's oder eines wirksamen Fragmentes davon gekoppelt ist, oder
VIP-NHCH₂CH₃
VIP-NH(CH₂)₃CH₃ oder
VIP-NH(CH₂)₇CH₃
dargestellt wird, wobei die hydrophobe Einheit durch das C-Ende des VIP's gekoppelt ist.

7. Konjugat nach Anspruch 1 der Formel

CH3(CH2)16-CO-His-Ser-Asp-Ala-Val-Phe-Thr-Asp-Asn-Tyr-Thr-
Arg-Leu-Arg-Lys-Gln-Met-Ala-Val-Lys-Lys-Tyr-Leu-Asn-Ser-
Ile-Leu-Asn-NH2,
CH3(CH2)16-CO-Thr-Asp-Asn-Tyr-Thr-Arg-Leu-Arg-Lys-Gln-Met-
Ala-Val-Lys-Lys-Tyr-Leu-Asn-Ser-Ile-Leu-Asn-NH2, oder
CH3(CH2)16-CO-Gln-Met-Ala-Val-Lys-Lys-Tyr-Leu-Asn-Ser-Ile-
Leu-Asn-NH2.

8. Transdermales Arzneimittel, das zur Behandlung von Impotenz bei Männern verwendbar ist, umfassend eine physiologisch wirksame Menge eines Konjugats nach einem der Ansprüche 1 bis 7, kombiniert mit einem pharmazeutisch verträglichen Träger.

9. Mittel nach Anspruch 8, wobei das Konjugat Stearyl-VIP ist, und der Träger DMSO umfaßt.

10. Verfahren zur Herstellung eines Konjugats des vasoaktiven intestinalen Peptids (VIP) oder eines wirksamen Fragments davon, gekoppelt an eine hydrophobe Einheit, wobei das wirksame Fragment derart wirksam ist, daß die Injektion des Fragments eine Erektion beim Mann induziert, das die Kopplung einer langkettigen aliphatischen Carbonsäure an das N-Ende der Peptidkette oder die Kopplung eines langkettigen aliphatischen Amins an das C-Ende der Peptidkette umfaßt.

11. Verfahren nach Anspruch 10, wobei die aliphatische Carbonsäure CH₃(CH₂)₁₆-COOH ist, und das Konjugat die Formel: 
CH3(CH2)16-CO-His-Ser-Asp-Ala-Val-Phe-Thr-Asp-Asn-Tyr-Thr-
Arg-Leu-Arg-Lys-Gln-Met-Ala-Val-Lys-Lys-Tyr-Leu-Asn-Ser-
Ile-Leu-Asn-NH2,
 
CH3(CH2)16-CO-Thr-Asp-Asn-Tyr-Thr-Arg-Leu-Arg-Lys-Gln-Met-
Ala-Val-Lys-Lys-Tyr-Leu-Asn-Ser-Ile-Leu-Asn-NH2, oder
CH3(CH2)16-CO-Gln-Met-Ala-Val-Lys-Lys-Tyr-Leu-Asn-Ser-Ile-
Leu-Asn-NH2
 aufweist.

12. Verfahren nach Anspruch 10 oder 11, wobei die Kopplung der aliphatischen Carbonsäure an das N-Ende der Peptidkette die Kopplung von Stearinsäure an das N-Ende der Peptidkette unter Verwendung von N,N'-Dicyclohexylcarbodiimid und 1-Hydroxylbenzotriazol als Kopplungsmittel umfaßt.

## Patentansprüche (Patentansprüche für folgende(n) Vertragsstaat(en): ES)

1. Verfahren zur Herstellung eines Konjugats des vasoaktiven intestinalen Peptids (VIP) oder eines wirksamen Fragments davon, gekoppelt an eine hydrophobe Einheit, wobei das wirksame Fragment derart wirksam ist, daß die Injektion des Fragments eine Erektion beim Mann induziert, das die Kopplung einer langkettigen aliphatischen Carbonsäure an das N-Ende der Peptidkette oder die Kopplung eines langkettigen aliphatischen Amins an das C-Ende der Peptidkette umfaßt.

2. Verfahren nach Anspruch 1, wobei die aliphatische Carbonsäure die Formel CH₃(CH₂)ₙ-COOH aufweist, wobei n eine ganze Zahl von 1 bis 16 ist.

3. Verfahren nach Anspruch 1, wobei das aliphatische Amin ein Amin der Formel CH₃(CH₂)ₘNH₂ ist, wobei m eine ganze Zahl von 1 bis 12 ist.

4. Verfahren nach einem der Ansprüche 1 bis 3, wobei das wirksame Fragment des VIP's VIP₇₋₂₈ oder VIP₁₆₋₂₈ ist.

5. Verfahren nach Anspruch 1, wobei die aliphatische Carbonsäure CH₃(CH₂)₆-COOH ist, und das Konjugat CH₃(CH₂)₆CO-VIP ist.

6. Verfahren nach Anspruch 1, wobei die aliphatische Carbonsäure CH₃(CH₂)₁₆-COOH ist, und das Konjugat die Formel: 
CH3(CH2)16-CO-His-Ser-Asp-Ala-Val-Phe-Thr-Asp-Asn-Tyr-Thr-
Arg-Leu-Arg-Lys-Gln-Met-Ala-Val-Lys-Lys-Tyr-Leu-Asn-Ser-
Ile-Leu-Asn-NH2,
CH3(CH2)16-CO-Thr-Asp-Asn-Tyr-Thr-Arg-Leu-Arg-Lys-Gln-Met-
Ala-Val-Lys-Lys-Tyr-Leu-Asn-Ser-Ile-Leu-Asn-NH2, oder
CH3(CH2)16-CO-Gln-Met-Ala-Val-Lys-Lys-Tyr-Leu-Asn-Ser-Ile-
Leu-Asn-NH2
 aufweist.

7. Verfahren nach einem der Ansprüche 1, 2, 4 oder 6, wobei die Kopplung der aliphatischen Carbonsäure an das N-Ende der Peptidkette die Kopplung von Stearinsäure an das N-Ende der Peptidkette unter Verwendung von N,N'-Dicyclohexylcarbodiimid und 1-Hydroxylbenzotriazol als Kopplungsmittel umfaßt.

8. Verfahren zur Herstellung eines transdermalen Arzneimittels, das die Kombination einer physiologisch wirksamen Menge eines nach einem der Ansprüche 1 bis 7 hergestellten Konjugats mit einem pharmazeutisch verträglichen Träger umfaßt.

9. Verfahren nach Anspruch 8, wobei das Konjugat Stearyl-VIP ist, und der Träger DMSO umfaßt.

## Revendications (Revendications pour l'(les) Etat(s) contractant(s) suivant(s): AT, BE, CH, DE, FR, GB, GR, IT, LI, LU, NL, SE)

1. Conjugué de peptide vaso-actif intestinal (VIP) ou d'un fragment actif de celui-ci, ledit fragment actif ayant une activité telle que son injection provoque une érection chez l'homme, associé à un motif hydrophobe.

2. Conjugué selon la revendication 1, dans lequel le motif hydrophobe est un motif aliphatique ou un motif de formule : 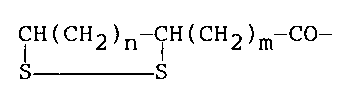 où n est un nombre entier valant de 2 à 6, et m est un nombre entier valant de 2 à 12, et est attaché à l'extrémité N-terminale de la molécule de VIP ou d'un fragment actif de celui-ci.

3. Conjugué selon la revendication 1 ou 2, dans lequel un motif hydrophobe est un motif de formule CH₃(CH₂)ₙ-CO-, où n est un nombre entier valant de 1 à 16, attaché à l'extrémité N-terminale de la molécule de VIP ou d'un fragment actif de celui-ci.

4. Conjugué selon la revendication 1, dans lequel le motif hydrophobe est un motif de formule CH₃(CH₂)ₘ-NH-, où m est un nombre entier valant de 1 à 12, attaché à l'extrémité C-terminale de la molécule de VIP ou d'un fragment actif de celui-ci.

5. Conjugué selon l'une quelconque des revendications 1 à 4, dans lequel le fragment actif de VIP est VIP₇₋₂₈ ou VIP₁₆₋₂₈.

6. Conjugué selon la revendication 1, représenté par la formule :
CH₃(CH₂)₆-CO-VIP ou 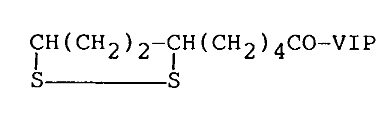
où le motif hydrophobe est couplé par l'intermédiaire de l'extrémité N-terminale du VIP, ou un fragment actif de celui-ci, ou
VIP-NHCH₂CH₃,
VIP-NH(CH₂)₃CH₃ ou
VIP-NH(CH₂)₇CH₃,
où le motif hydrophobe est couplé par l'intermédiaire de l'extrémité C-terminale du VIP.

7. Conjugué selon la revendication 1, répondant à la formule :

CH3(CH2)16-CO-His-Ser-Asp-Ala-Val-Phe-Thr-Asp-Asn-Tyr-Thr-
Arg-Leu-Arg-Lys-Gln-Met-Ala-Val-Lys-Lys-Tyr-Leu-Asn-Ser-
Ile-Leu-Asn-NH2,
CH3(CH2)16-CO-Thr-Asp-Asn-Tyr-Thr-Arg-Leu-Arg-Lys-Gln-Met-
Ala-Val-Lys-Lys-Tyr-Leu-Asn-Ser-Ile-Leu-Asn-NH2,
ou
CH3(CH2)16-CO-Gln-Met-Ala-Val-Lys-Lys-Tyr-Leu-Asn-Ser-Ile-
Leu-Asn-NH2.

8. Composition pharmaceutique transdermique pouvant être utilisée pour le traitement de l'impuissance masculine, comprenant une quantité physiologiquement efficace d'un conjugué selon l'une quelconque des revendications 1 à 7, combiné avec un excipient pharmaceutiquement acceptable.

9. Composition selon la revendication 8, dans laquelle le conjugué est le stéaryle-VIP, et l'excipient est constitué de DMSO.

10. Procédé de préparation d'un conjugué de peptide vaso-actif intestinal (VIP) ou d'un fragment actif de celui-ci, ledit fragment actif ayant une activité telle que son injection provoque une érection chez l'homme, associé à un motif hydrophobe, comprenant le couplage d'un acide carboxylique aliphatique à chaîne longue à l'extrémité N-terminale de la chaîne peptidique ou le couplage d'une amine aliphatique à chaîne longue à l'extrémité C-terminale de la chaîne peptidique.

11. Procédé selon la revendication 10, dans lequel l'acide carboxylique aliphatique est CH₃(CH₂)₁₆-COOH, et le conjugué répond à la formule :

CH3(CH2)16-CO-His-Ser-Asp-Ala-Val-Phe-Thr-Asp-Asn-Tyr-Thr-
Arg-Leu-Arg-Lys-Gln-Met-Ala-Val-Lys-Lys-Tyr-Leu-Asn-Ser-
Ile-Leu-Asn-NH2,
CH3(CH2)16-CO-Thr-Asp-Asn-Tyr-Thr-Arg-Leu-Arg-Lys-Gln-Met-
Ala-Val-Lys-Lys-Tyr-Leu-Asn-Ser-Ile-Leu-Asn-NH2,
ou
CH3(CH2)16-CO-Gln-Met-Ala-Val-Lys-Lys-Tyr-Leu-Asn-Ser-Ile-
Leu-Asn-NH2.

12. Procédé selon la revendication 10 ou 11, dans lequel le couplage de l'acide carboxylique aliphatique à l'extrémité N-terminale de la chaîne peptidique comprend le couplage de l'acide stéarique à l'extrémité N-terminale de la chaîne peptidique en utilisant le N,N'-dicyclohexylcarbodiimide et le 1-hydroxybenzotriazole comme agents de couplage.

## Revendications (Revendications pour l'(les) Etat(s) contractant(s) suivant(s): ES)

1. Procédé de préparation d'un conjugué de peptide vaso-actif intestinal (VIP), ou d'un fragment actif de celui-ci, ledit fragment actif ayant une activité telle que son injection provoque une érection chez l'homme, associé à un motif hydrophobe, comprenant le couplage d'un acide carboxylique aliphatique à chaîne longue à l'extrémité N-terminale de la chaîne peptidique ou le couplage d'une amine aliphatique à chaîne longue à l'extrémité C-terminale de la chaîne peptidique.

2. Procédé selon la revendication 1, dans lequel l'acide carboxylique aliphatique a la formule CH₃(CH₂)ₙ-COOH, où n est nombre entier valant de 1 à 16.

3. Procédé selon la revendication 1, dans lequel l'amine aliphatique est une amine de formule CH₃(CH₂)ₘNH₂, où m est un nombre entier valant de 1 à 12.

4. Procédé selon l'une quelconque des revendications 1 à 3, dans lequel le fragment actif de VIP est VIP₇₋₂₈ ou VIP₁₆₋₂₈.

5. Procédé selon la revendication 1, dans lequel l'acide carboxylique aliphatique est CH₃(CH₂)₆-COOH, et le conjugué est CH₃(CH₂)₆CO-VIP.

6. Procédé selon la revendication 1, dans lequel l'acide carboxylique aliphatique est CH₃(CH₂)₁₆-COOH, et le conjugué répond à la formule :

CH3(CH2)16-CO-His-Ser-Asp-Ala-Val-Phe-Thr-Asp-Asn-Tyr-Thr-
Arg-Leu-Arg-Lys-Gln-Met-Ala-Val-Lys-Lys-Tyr-Leu-Asn-Ser-
Ile-Leu-Asn-NH2,
CH3(CH2)16-CO-Thr-Asp-Asn-Tyr-Thr-Arg-Leu-Arg-Lys-Gln-Met-
Ala-Val-Lys-Lys-Tyr-Leu-Asn-Ser-Ile-Leu-Asn-NH2,
ou
CH3(CH2)16-CO-Gln-Met-Ala-Val-Lys-Lys-Tyr-Leu-Asn-Ser-Ile-
Leu-Asn-NH2.

7. Procédé selon l'une quelconque des revendications 1, 2, 4 ou 6, dans lequel le couplage de l'acide carboxylique aliphatique à l'extrémité N-terminale de la chaîne peptidique comprend le couplage de l'acide stéarique à l'extrémité N-terminale de la chaîne peptidique en utilisant le N,N'-dicyclohexylcarbodiimide et le 1-hydroxybenzotriazole comme agents de couplage.

8. Procédé de préparation d'une composition pharmaceutique transdermique, comprenant la combinaison d'une quantité physiologiquement efficace d'un conjugué préparé selon l'une quelconque des revendications 1 à 7 avec un excipient pharmaceutiquement acceptable.

9. Procédé selon la revendication 8, dans lequel le conjugué est le stéaryle-VIP, et l'excipient est constitué de DMSO.
